# EUROPÄISCHE PATENTSCHRIFT

(11) **EP 1 818 067 B1**
(45) Veröffentlichungstag und Bekanntmachung des Hinweises auf die Patenterteilung: **12.11.2008**
(21) Anmeldenummer: 07101346.0
(22) Anmeldetag: 29.01.2007
(51) Int. Cl.: A61M 1/06

(54) **Milchpumpe**
Milk pump
Tire-lait

(30) Priorität: 09.06.2006 CH 9382006; 13.02.2006 DE 102006006417
(43) Veröffentlichungstag der Anmeldung: 15.08.2007
(73) Patentinhaber: Trimed AG, 9495 Triesen (LI)
(72) Erfinder: Nüesch, Hansueli, 5453 Remetschwil (CH)
(74) Vertreter: Stocker, Kurt

(56) Entgegenhaltungen:
- EP-A2- 0 733 376
- WO-A-03/013628
- WO-A-20/04058330
- WO-A-20/06000292
- WO-A-20/06117352
- WO-A-20/07017968
- US-A- 5 358 476
- US-A- 2001 038 799
- US-A1- 2002 072 702

## Beschreibung

Die Erfindung bezieht sich auf eine Milchpumpe nach dem Oberbegriff des Anspruches 1.

Eine derartige Milchpumpe ist beispielsweise aus der US-A-4,813,932, aber auch aus der EP-1 231 955 bekannt geworden. In beiden Fällen wird ein Kolben innerhalb des Hubraumes von einem Betätigungshebel bewegt. Besonders berufstätige Mütter pumpen so Muttermilch ab, um sie dem Säugling während des Tages, während welcher Zeit das Kind von einer Amme oder einer Verwandten betreut wird, zur Verfügung zu stellen.

Es ist bekannt, dass berufstätige Mütter einer grossen Stressbelastung ausgesetzt sind. Das Abpumpen der Muttermilch nimmt eine gewisse Zeit in Anspruch, was die morgendliche Stress-Situation noch verschärft. Obgleich nun aber Handpumpen der oben genannten Art preislich relativ günstig sind, verzichten manche Frauen darauf und wählen lieber die teureren Motorpumpen. Als Grund wird vielfach die rasche Ermüdung der Hand angegeben.

Eine solche Ermüdung stellt sich besonders dann ein, wenn die Pumpe kurzhubig ist und daher viele Pumpbewegungen nötig sind, um einen angemessenen Saugeffekt zu erzielen. Dies gilt besonders etwa für Konstruktionen mit relativ kurzen Membranen, beispielsweise der einen Kolben abdichtenden Rollmembrane nach der WO 2004/058330, der US-A-2002/0072702 (vgl. Fig. 17) oder der EP-0 733 376, deren Hub eben ziemlich begrenzt ist, so dass sie öfter betätigt werden müssen, um dasselbe Ergebnis wie etwa eine Kolbenpumpe zu erreichen. Zudem sind solche Membrane auf Zug relativ empfindlich und können leicht reissen, weil sie im allgemeinen entgegen ihrer Eigenelastizität bewegt werden.

Da das Überlaufen der Milch in den Pumpraurn verhindert werden muss, ist es auch bekannt geworden (vgl. WO 2006/000292), eine Membrane über den gesamten Saugquerschnitt als Dichtung einzusetzen und diese Membrane indirekt durch einen Kolben und dessen Saugwirkung zu bewegen. Allerdings ist diese Konstruktion ziemlich platzaufwendig und hat auch den Nachteil, dass die Kolbenbewegung mit der Membranbewegung synchron ablaufen muss; wird etwa die Membrane beim Einsetzen in die Pumpe (was zum Waschen nach Gebrauch erforderlich ist) etwas gequetscht eingesetzt, wogegen der Kolben den ersten Hub in voller Länge ausführt, dann kann eine effiziente und leichte Pumparbeit nicht mehr geleistet werden.

Der vorliegenden Erfindung liegt nun in einem ersten Schritt die Aufgabe zugrunde, welche sich der Erfinder gestellt hat, nämlich die Ursache der leichten Ermüdbarkeit aufzuspüren und Massnahmen dagegen zu ergreifen.

Bei einer Untersuchung einer grösseren Anzahl solcher und ähnlicher Handpumpen wurde festgestellt, dass ein Gutteil der von Hand eingebrachten Energie durch die Reibung der Kolben an der Wandung des Hubraumes absorbiert wird. Denn es ist klar, dass die Pumpenkolben an ihrem Umfang gegenüber der Wandung abgedichtet sein müssen, wenn nicht ein Teil der Saugwirkung verloren gehen soll, was dann zu einem vermehrten Pumpaufwand führen würde.

Im Zuge der Untersuchung tauchte aber auch noch ein anderer Grund für eine erschwerte Betätigung des Pumpenkolbens auf: Schon relativ kleine Toleranzen bei der Ausformung des Hubraumes oder mechanisch oder thermisch bedingte Verformungen erschweren die Betätigung erheblich. Dies ist bei einem Hubraum mit gerader Längsachse, wie in der genannten US-A-4,813,932 zwar weniger der Fall, doch ergibt sich dort ein grösserer Widerstand durch die Reibung eines Betätigungsansatzes an einer mit dem Kolben verbundenen Betätigungsfläche. Dies Reibung tritt dadurch auf, dass der Betätigungshebel zwar eine Schwenkbewegung ausführt, diese aber - bei geradem Hubraum - in eine geradlinige Bewegung umgesetzt werden muss.

Daher hat die EP-1 231 955 einen bogenförmigen Hubraum vorgeschlagen, der gewissermassen die Hüllfläche der Kolbenbewegung ist. Hier aber verschärft sich das Problem: Abgesehen davon, dass dadurch die Entformung des Spritzgussteils erschwert wird, setzt eine solche Konstruktion eine erhöhte Präzision voraus: denn wenn nicht der Mittelpunkt der Krümmung des bogenförmigen Hubraumes genau mit dem Mittelpunkt der bogenförmigen Kolbenbewegung übereinstimmt, kommt es zu einer erhöhten Reibung der Kolbendichtung an der Wandung des Hubraumes, was die Betätigung der Milchpumpe arg behindert. Eine solche Abweichung kann sich aber aus vielerlei Gründen ergeben: durch unvermeidliche Toleranzen beim Spritzgiessen, durch mechanische oder thermische Einflüsse bzw. Belastungen sowie einfach durch Abnützungserscheinungen. Die dadurch hervorgerufene erhöhte Reibung bewirkt dann schliesslich die raschere Ermüdung der Hand beim Pumpen.

Natürlich kann eine solche Ermüdung leicht vermieden werden, wenn an Stelle einer Handpumpe eine Motorpumpe verwendet wird, zumal die Motoren ja im allgemeinen sowieso überdimensioniert sein müssen, um solche Toleranzen leicht überwinden zu können. Dort stellt sich also das oben angesprochene Problem gar nicht. Allerdings sind eben solche Pumpen stets mit einem höheren Investitionsaufwand verbunden, der sich angesichts der meist gegebenen Kurzlebigkeit des Bedarfes gar nicht lohnt.

Die WO 03/013628 zeigt eine Milchpumpe mit den Oberbegriffsmerkmalen des Anspruches 1. Ebenso, wie bei vielen anderen der oben genannten Pumpen, ergibt sich das Problem, dass im entspannten Zustand die Membrane, mindestens mit einem Teil ihrer Aussenfläche, an einer Innenfläche des das sogenannte "Horn" 4 aufweisenden Teil des Körpers 1 anliegt. Da diese Innenflächen aber von Milch durchströmt sind, besteht die Gefahr, dass die Aussenflächen der Membrane an den Innenflächen des Körpers 1 "ankleben", das Pumpen behindern bzw. erschweren (und damit zur Ermüdung beitragen), mit einem Verunreinigungen bildenden Film überzogen werden etc.

Der Erfindung liegt daher in einem zweiten Schritt die Aufgabe zugrunde, eine Milchpumpe der eingangs genannten Art so auszustatten, dass ihr Betrieb weniger leicht eine Ermüdung verursacht. Dies gelingt erfindungsgemäss durch die kennzeichnenden Merkmale des Anspruches 1.

Wie oben bereits erwähnt, wurden Membrane bereits in verschiedener Form für Milchpumpen angewandt. Beispielsweise schlägt die DE-C-729 251, die DE-U-82 14 315 oder die EP-0 000 339, aber auch die US-A-2002/0072702 (Fig. 1) die Verwendung einer ballonartigen Membrane als Pumpe vor, die nicht in einem Hubraum untergebracht ist. Solche Pumpballone sind allerdings nicht ohne Mühe zu betätigen.

Aus der WO 92/00111 ist auch ein Schleimextraktor bekannt geworden, bei dem als Pumpmembran ein vom Daumen betätigbarer Faltenbalg angewendet wird. Nun sagt ja schon der Name "Faltenbalg", dass hier der Membranteil ihm eine gewisse Elastizität verleihende Einziehungen, nämlich die Falten, aufweisen muss. Dadurch wird natürlich das mögliche Pumpvolumen stark begrenzt. Das ist ja auch bei Schleim, der im allgemeinen nur in geringen Mengen anfällt, nicht so schlimm, löst aber bei Milchpumpen das oben angesprochene Problem nicht, weil dadurch häufigere Pumpbewegungen durchzuführen sind, um praktisch dasselbe Ergebnis wie bei einer Kolbenpumpe zu erhalten. Ausserdem ist die dauernde Belastung des Daumes auch ermüdend.

Durch die erfindungsgemässe Ausgestaltung der Membrane ist zunächst einmal gesichert, dass Reibungsverluste praktisch nicht auftreten, weil eine an der Wandung reibende Kolbendichtung nicht vorgesehen ist und die Membrane innerhalb des Hubraumes so viel Freiraum besitzt, dass sie auch gegen die Wandung hin beweglich ist. Dadurch, dass die Membrane ausser einem sich quer zum Hubraum erstreckenden Abschnitt auch noch einen - im durch den Hubraum gestreckten Zustand - sich etwa in Umfangsrichtung der Wandung erstreckenden Abschnitt aufweist, d.h. also etwa becherförmig ausgebildet ist, wird ein relativ grosser Hub ermöglicht, so dass das Abpumpen der Muttermilch relativ rasch und mit wenig Mühe von statten gehen kann.

Bevorzugt ist die Ausbildung der Betätigungseinrichtung als zweiarmiger, in einem Mittelbereich schwenkbar gelagerter Hebel, weil diese Art der Betätigungseinrichtung mechanisch einfach ist und überdies auch noch die Erleichterung der Hebelübersetzung der aufgewendeten Kraft ergibt.

Bei bekannten Einrichtungen ist im allgemeinen eine Rückholfeder zum Rückholen der Betätigungseinrichtung in eine vorbestimmte Ausgangslage vorgesehen. An sich wäre dies nicht unbedingt erforderlich, denn es wäre, wie bei einer Schere, möglich, Betätigungsösen zum Durchstecken wenigstens eines Fingers (vgl. die Öse oder Schlaufe 58 in der WO 95/18639) vorzusehen und auf eine Rückholeinrichtung, die an sich wieder in beliebiger Weise ausgebildet sein mag, zu verzichten. Bevorzugt ist aber auch im Rahmen der vorliegenden Erfindung, eine Rückholeinrichtung einzusetzen, wenngleich diese Aufgabe nach einer bevorzugten Ausführung der Erfindung gleich von der becherförmigen Membrane selbst übernommen werden kann, wie noch beschrieben wird.

Ein besonders günstiger Hub der Membran wird ermöglicht, wenn die Membrane etwa topfförmig mit einer Bodenfläche und mindestens einer Umfangsfläche ausgebildet ist. Gerade eine solche Membrane, aber auch jede andere in den Rahmen dieser Erfindung fallende, wird vorteilhaft in ihrer Bewegung geführt. Zu diesem Zweck ist es bevorzugt, wenn im Mittelbereich an dem sich quer zum Hubraum erstreckenden Membranabschnitt ein mit dem Hebel verbundener Führungsteil befestigt ist. Dieser, vorzugsweise scheibenförmige, Führungsteil ist zweckmässig gegenüber der kleinsten Querabmessung des Hubraumes um wenigstens 15%, vorzugsweise wenigstens 20%, insbesondere wenigstens 30% kleiner, beispielsweise etwa 35% kleiner, so dass mit Sicherheit Reibungswiderstände vermieden sind.

Eine weitere vorteilhafte Massnahme liegt darin, dass der Hubraum eine gerade Längsachse und/oder eine sich gerade erstreckende, gegebenenfalls aber auch eine von der genannten Öffnung weg divergierende, Wandung aufweist. Damit wird seine Entformung erleichtert. Ausserdem wird, wie unten noch deutlich wird, so ein Raum geschaffen, in dem sich tatsächlich die Membrane bzw. ihr Führungsteil mit einer Querkomponente bewegen kann (etwa, wenn trotz der im wesentlichen linearen Erstreckung des Hubraumes eine bogenförmige Bewegung der Membrane und/oder ihrer Führung bewirkt wird), ohne dass sich dadurch Reibungsverluste ergeben, die zur Ermüdung der pumpenden Hand führen können.

Gemäss einer Weiterbildung der Erfindung weist der Hubraum einen von einem zylindrischen Querschnitt abweichenden Querschnitt auf. Dies kann beispielsweise ein ovaler Querschnitt sein, insbesondere ein solcher, dessen Oval eine im wesentlichen vertikale längere Ovalachse aufweist. Diese letztere Ausbildung ist besonders dann vorteilhaft, wenn etwa der Hubraum eine geradlinige Achse besitzt und der Führungsteil für die Membrane eine Schwenkbewegung ausführt. Besonders bevorzugt ist aber aus mechanischen Gründen eine Ausführung mit einem etwa viereckigen Querschnitt mit abgerundeten Ecken.

Eine besonders einfache und kostengünstige Gestaltung ergibt sich, wenn im Rahmen der Erfindung die becherförmige Membrane wenigstens annähernd kalottenförmig ausgebildet ist und mit dieser Form selbst die Rückholeinrichtung bildet. An sich wäre es ja denkbar, diese Rückholeinrichtung etwa durch eine Feder noch zu ergänzen, doch ist dies im allgemeinen nicht nötig. Diese Ausbildung führt zwar zu einer unterschiedlichen Saugwirkung über den Betätigungsweg der Betätigungseinrichtung, doch wird dies für einfachere Pumpenausführungen nicht störend sein.

In einem solchen Falle ist es aber vorteilhaft, wenn der Hubraum einen etwa kalottenförmigen, die Membrane aufnehmenden Querschnitt aufweist. In der Praxis wird dann die Kalottenkrümmung beider Teile vorteilhaft gleich dimensioniert sein, so dass sie einander ergänzen und damit eine möglichst grosse Saugwirkung bei der Betätigung gesichert ist.

Weitere Einzelheiten der Erfindung ergeben sich an Hand der nachfolgenden Beschreibung eines in der Zeichnung dargestellten bevorzugten Ausführungsbeispieles. Es zeigen:
Fig. 1 eine Perspektivansicht einer erfindungsgemässen Milchpumpe;
Fig. 2 einen Vertikalschnitt durch die Pumpe nach Fig. 1; und die
Fig. 3 eine explodierte Darstellung des Hubraumes und der darin untergebrachten Teile;

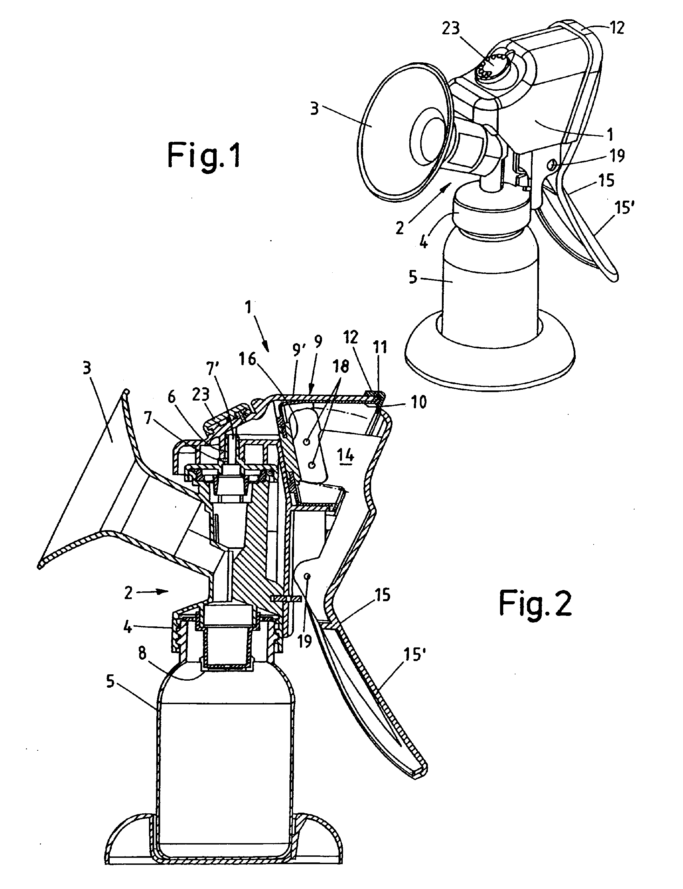

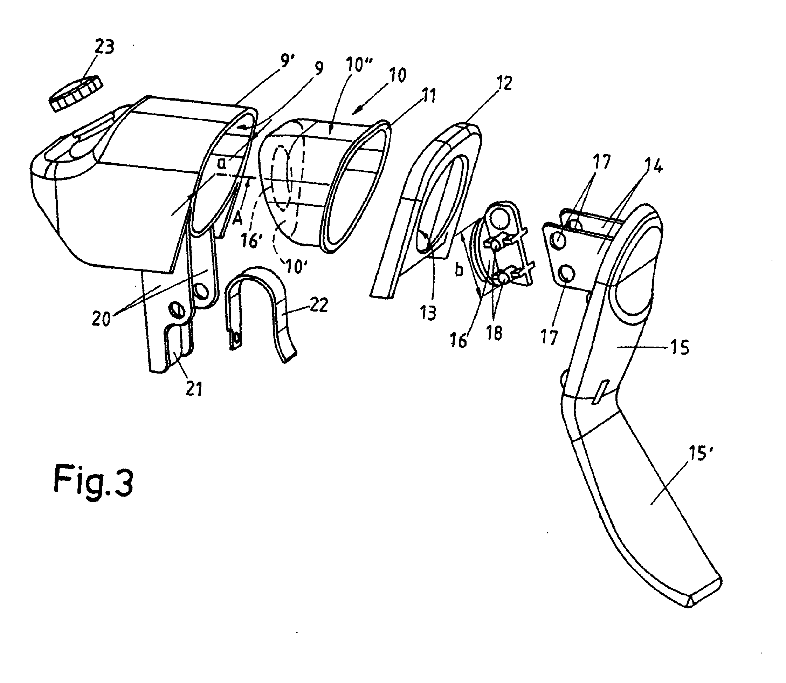

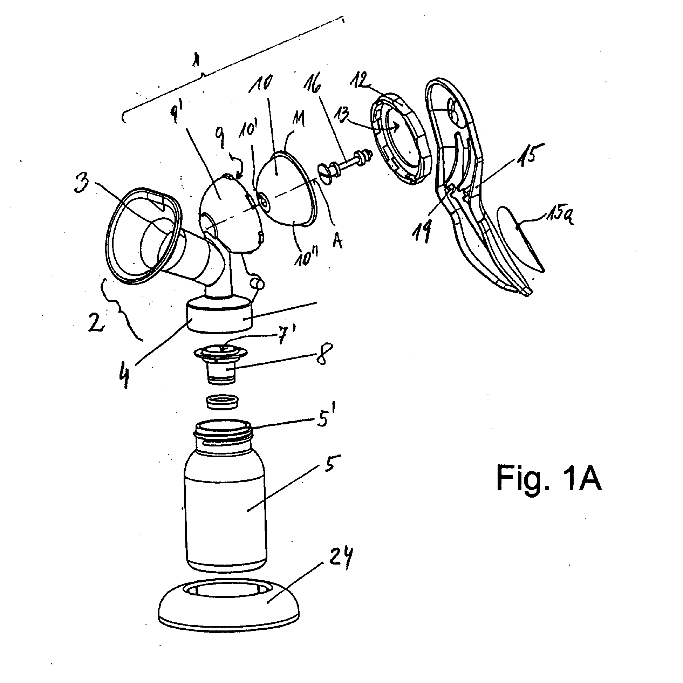

Fig. 1A eine der Fig. 3 ähnliche explodierte Darstellung einer vorteilhaften Ausführungsform der Milchpumpe mit dem Hubraum und den damit zusammenwirkenden Teilen; und

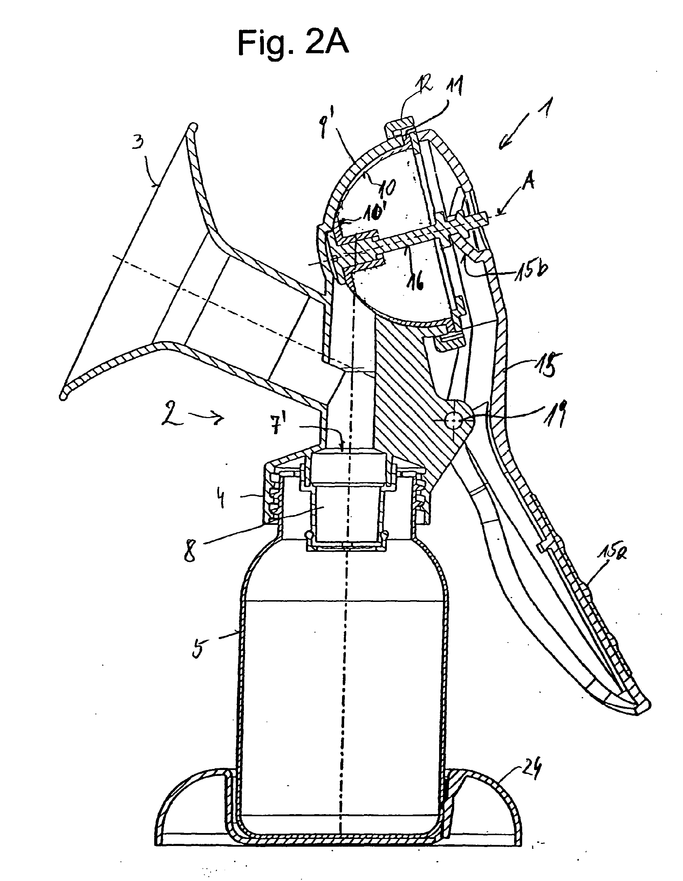

Fig. 2A einen Längsschnitt durch die zusammengebaute Milchpumpe nach Fig. 1A.

Die in den Fig. 1 und 2 dargestellte Milchpumpe weist einen Oberteil 1 (Handpumpeneinheit) auf, der auf einen Verbindungsteil 2 aufgesteckt ist (bei 6, 7, an der Verbindungsöffnung 7'). Der Verbindungsteil 2 umfasst einerseits ein Brustansatzstück 3 oder Horn, anderseits einen Schraubteil 4 mit dem die Pumpe auf einen Milchsammelbehälter 5 aufschraubbar ist. Zwischen Milchsammelbehälter 5 und dem Verbindungsteil 2 liegt in bekannter Weise ein Rückschlagventil 8 (Fig. 2). An der Oberseite mag ein bekannter Reglerknopf 23 angebracht sein, der die Zufuhr von Falschluft regelt und damit auch die Mühe bei der Pumpbetätigung. Eine besonders bevorzugte Ausführung ist beispielsweise in den US-A-6,042,560 und 6,290,671 beschrieben, deren Inhalt hier durch Bezugnahme als geoffenbart gelten soll.

Innerhalb des Oberteils 1 ist ein, am besten aus Fig. 3 ersichtlicher Hubraum 9 vorgesehen, der von einer Wandung 9' umgeben ist. Die Wandung 9' hat, wie besonders Fig. 3 zeigt, einen im wesentlichen viereckigen Querschnitt mit abgerundeten Ecken. In disen Hubraum 9 ist eine etwa topfförmige Membrane 10 eingesetzt und mit einem ihren Rand 11 festhaltenden Rahmen 12 an einem Ende der den Hubraum 9 begrenzenden Wandung 9' befestigt.

Der Rahmen 12 besitzt eine Durchlassöffnung 13, durch welche ein an zwei Vorsprüngen 14 eines zweiarmigen Betätigungshebels 15 befestigtes, etwa scheibenförmiges, Führungsschild 16 angebracht ist. Hierzu weisen die Vorsprünge 14 gemäss Fig. 3 Löcher 17 auf, in welche Stifte oder Vorsprünge 18 am Führungsschild 16 einsetzbar und/oder ein-schnappbar sind.

Fig. 2 veranschaulicht strich-punktiert die Bewegung des Führungsschildes 16, an dem die etwa topfförmige Membrane 10 befestigt ist. Zu diesem Zweck weist die Membrane 10 einen sich quer zum Hubraum 9 erstreckenden Abschnitt 10' (strichliert in Fig. 2) mit einer das Führungsschild 16 aufnehmenden Öffnung 16' (vgl. Fig. 2, 3) und einen - im durch den Hubraum 9 gestreckten Zustand (vgl. Fig. 2, 3) - sich etwa in Umfangsrichtung der Wandung 9' erstreckenden Abschnitt 10" auf. Der Abschnitt 10' bildet also sozusagen die Bodenfläche der topfförmigen Membrane 10, wogegen der Abschnitt 10" die (mindestens eine) Umfangsfläche bildet.

Da der Hubraum 9 eine geradlinige Achse A besitzt, das Führungsschild 16 dagegen mit dem Hebel 15 verbunden ist und mit diesem um eine Schwenkachse 19 (Fig. 1, 2) schwenkt, ergibt sich - wie aus Fig. 2 strich-punktiert ersichtlich - bei Druck auf das untere Ende des Hebels 15 eine Bewegung im Sinne der strich-punktierten Linien, was an der Oberseite des Hubraumes 9 zu einer geringfügigen Wegbewegung des Schildes 16 von der Wandung 9', an der Unterseite dagegen eine Annäherung bewirkt. Das bedeutet, dass die Membrane 10 auch gegen die Wandung 9' hin beweglich ist. Aber auch im Falle einer Übereinstimmung der Bewegungsachse des Führungsschildes 16 mit der Längsachse A (etwa beide gekrümmt, wie beim Stande der Technik oder beide geradlinig) ist der Unterschied zwischen der Querdimension des Führungsschildes 16 zur lichten Weite des Hubraumes 9 derart gestaltet, dass eine Bewegung der Membrane 10 zur Wandung mindestens möglich wäre. In diesem Sinne ist das betreffende Merkmal im Anspruch 1 zu verstehen.

Um eine solche Querbewegung des Führungsschildes 16 gegenüber der Wandung zu ermöglichen, ist der, vorzugsweise scheibenförmige, Führungsteil 16 mit seiner Querabmessung b gegenüber der kleinsten Querabmessung a (Fig. 3) des Hubraumes 9 um wenigstens 15%, vorzugsweise wenigstens 20%, insbesondere wenigstens 30% kleiner ist, beispielsweise etwa 35%, kleiner. Dies lässt Platz für die Bewegung der Membrane 10 und vermeidet jegliche Reibung, welche die Betätigung der Membrane 10 bei ihrer Hin- und Herbewegung innerhalb des Hubraumes 9 erschweren könnte.

Wäre am unteren Betätigungsende 15' des Hebels 15 ein Ring zum Durchstecken eines Fingers (oder ein Bügel für mehrere Finger) angebracht, dann bedürfte es keiner Rückholeinrichtung. Bevorzugt ist jedoch zwischen zwei Haltewangen 20, sich an einer Querwand 21 abstützend, eine einfach als Blattfeder 22 ausgebildete Rückholfeder vorgesehen, deren dem Hebel 15 zugekehrte Seite sich an diesem Hebel abstützt. Damit wird der Hebel und die Membrane stets in eine vorbestimmte Ausgangslage rückgeholt und die Bedienungsperson braucht also nur auf das Ende 15' zu drücken, um die Membrane 10 zu betätigen, d.h. zusammenzuziehen und zu strecken.

Die in den Fig. 1A und 2A dargestellte Milchpumpe steht mit ihrem Milchbehälter 5 zweckmässig in einem die Standfläche vergrössernden Fussteil 24. Innerhalb des Oberteils 1 ist wiederum ein Hubraum 9 vorgesehen, der von einer Wandung 9' umgeben ist. Die Wandung 9' hat hier vorzugsweise eine im wesentlichen kugelkalottenartige Form. In diesen Hubraum 9 ist eine vorteilhaft komplementär geformte kalottenförmige Membrane 10 aus elastischem Material eingesetzt und mit einem ihren Rand 11 festhaltenden Rahmen 12 an einem Ende der den Hubraum 9 begrenzenden Wandung 9' befestigt. Die Kalottenkrümmung von Hubraum 9 und Membrane 10 sind also im wesentlichen hinsichtlich Umfang, Krümmung und axialer Länge in Richtung der Achse A gleich dimensioniert.

Anderseits ist aus Fig. 2A ersichtlich, dass die Dicke der Membrane 10, in axialer Richtung (Achse A) variiert. Damit lässt sich einerseits die Federcharakteristik beeinflussen, anderseits wird durch die dargestellte Dickenvariation, nämlich mit der geringsten Dicke im mittleren Bereich 10' und der grössten Dicke im Bereiche des Randes 11, ein gleichmässiges Falten der Membrane bei Betätigung des Hebelarmes 15a gesichert. Diese Dickenvariation könnte an sich auch stufenförmig verlaufen, ist aber bevorzugt gleichmässig verlaufend, wie in Fig. 2A dargestellt. Andere Dickenvariationen sind im Rahmen der Erfindung durchaus möglich, etwa die Einarbeitung von verdickten, sich um die Achse A herum erstreckenden Ringen und/oder Nuten. Selbstverständlich wäre es auch möglich, die etwa topfförmige Membrane nach den Fig. 1 bis 3 mit unterschiedlichen Dicken, Ringen oder Nuten zu versehen.

Der Rahmen 12 besitzt wiederum eine Durchlassöffnung 13, durch welche ein an einem zweiarmigen, um eine Achse 19 schwenkbaren Betätigungshebel 15 mit Grifffläche 15a befestigter, etwa stiftförmiger Führungsteil 16 angebracht ist, dessen freies Ende mit10 dem sich quer zum Hubraum 9 und seiner Achse A erstreckenden, mittleren Abschnitt 10' verbunden ist (vgl. Fig. 2). Der Führungsteil 16 ist an einer Haltefläche 15b des Hebels 15 eingehängt, so dass er gegebenenfalls auch leicht demontierbar ist.

Mindestens im gestreckten Zustand nach den Fig. 1 und 2 erstreckt sich ein daran anschliessender Abschnitt 10" etwa in Umfangsrichtung der Wandung 9'. Auf diese Weise ist eine Bewegung der Membrane 10 zur Wandung 9' und von dieser weg möglich. Jedenfalls ist der Führungsteil 16 an der Stelle der Befestigung am mittleren Abschnitt 10' gegenüber der kleinsten Querabmessung des Hubraumes 9 bedeutend kleiner. Dies lässt Platz für die Bewegung der Membrane 10 und vermeidet jegliche Reibung, welche die Betätigung der Membrane 10 bei ihrer Hin- und Herbewegung innerhalb des Hubraumes 9 erschweren könnte.

Wäre am unteren Betätigungsende 15' des Hebels 15 ein Ring zum Durchstecken eines Fingers (oder ein Bügel für mehrere Finger) angebracht, dann bedürfte es keiner Rückholeinrichtung. Da aber die Membrane 10 notwendig eine gewisse Eigenelastizität besitzt und daher bestrebt ist, ihre Kalottenform zu erhalten, wirkt sie selbst als Rückholeinrichtung, so dass eine besondere Einrichtung hierfür entbehrlich ist.

## Patentansprüche

1. Milchpumpe mit einer Handpumpeneinheit (1) oberhalb einer zu einem Behälter (5) führenden Öffnung (7'), welche Einheit (1) einen im wesentlichen von wenigstens einer Wandung (9') umschlossenen, an ein Brustansatzstück (3) angeschlossenen oder anschliessbaren, eine Längsachse (A) definierenden Hubraum (9) und ein Saugorgan aufweist, das darin mittels eines manuelle betätigbaren Betätigungshebels (15), vorzugsweise eines zweiarmigen, in einem Mittelbereich (bei 19) schwenkbar gelagerten Hebels (15), hin- und herbewegbar ist, wobei vorzugsweise eine Rückholeinrichtung für die Betätigungseinrichtung (15) zum Rückholen in eine vorbestimmte Ausgangslage vorgesehen ist, wobei das Saugorgan von einer innerhalb des Hubraumes (9) gegen die Wandung (9') hin beweglichen, einen sich quer zum Hubraum (9) erstreckenden Abschnitt (10') und - im durch den Hubraum (9) gestreckten Zustand - sich etwa auch in Umfangsrichtung der Wandung (9') erstreckenden, somit etwa becherförmigen Membrane (10) mit Dickenvariationen gebildet ist, die von der Betätigungseinrichtung (15) innerhalb des Hubraumes (9) gleitfrei hin- und herbewegbar ist, **dadurch gekennzeichnet, dass** die Dickenvariationen der Membrane (10) die geringste Dicke im mittleren Bereich (10') und die grösste Dicke im Bereiche des Randes (11) aufweisen.

2. Milchpumpe nach Anspruch 1, **dadurch gekennzeichnet, dass** im Mittelbereich an dem sich quer zum Hubraum erstreckenden Membranabschnitt (10'), insbesondere in dessen Mitte, ein mit dem Hebel (15) verbundener Führungsteil (16) befestigt ist, und dass bevorzugt der, vorzugsweise scheibenförmige, Führungsteil (16) gegenüber der kleinsten Querabmessung (a) des Hubraumes (9) um wenigstens 15%, vorzugsweise wenigstens 20%, insbesondere wenigstens 30% kleiner ist, beispielsweise etwa 35%, kleiner ist.

3. Milchpumpe nach Anspruch 1 oder 2, **dadurch gekennzeichnet, dass** der Hubraum (9) eine gerade Längsachse (A) und/oder eine sich gerade erstreckende, gegebenenfalls aber auch eine von der genannten Öffnung (7') weg divergierende, Wandung (9') aufweist.

4. Milchpumpe nach einem der vorhergehenden Ansprüche, **dadurch gekennzeichnet, dass** der Hubraum (9) einen von einem zylindrischen Querschnitt abweichenden Querschnitt aufweist, insbesondere einen etwa viereckigen Querschnitt mit abgerundeten Ecken.

5. Milchpumpe nach einem der vorhergehenden Ansprüche, **dadurch gekennzeichnet, dass** die becherförmige Membrane (10) etwa topfförmig mit einer Bodenfläche (10') und mindestens einer Umfangsfläche (10") ausgebildet ist.

6. Milchpumpe nach einem der Ansprüche 1 bis 4, **dadurch gekennzeichnet, dass** die becherförmige Membrane (10) wenigstens annähernd kalottenförmig ausgebildet ist und mit dieser Form selbst die Rückholeinrichtung bildet.

7. Milchpumpe nach Anspruch 6, **dadurch gekennzeichnet, dass** der Hubraum (9) einen etwa kalottenförmigen, die Membrane (10) aufnehmenden Querschnitt aufweist, und dass vorzugsweise die Kalottenkrümmung beider Teile im wesentlichen gleich dimensioniert ist.

8. Milchpumpe nach einem der vorhergehenden Ansprüche, **dadurch gekennzeichnet, dass** die Dicke der Membrane (10) in Richtung ihrer Längsachse (A) variiert.

9. Milchpumpe nach Anspruch 8, **dadurch gekennzeichnet, dass,** bevorzugt im Falle einer kalottenförmigen Membrane (10), die Membrane in ihrem mittleren, mit der Betätigungseinrichtung (15) verbundenen Abschnitt (10') dünner als an ihrem Rande (11) ist und vorzugsweise eine im wesentlichen gleichmässig verlaufende Dickenvariation aufweist.

## Claims

1. Milk pump comprising a manual pump unity (1) above an opening (7') that leads to a receptacle (5), wherein said unity (1) includes a displacement space (9) enclosed by substantially at least one wall (9'), joined or to be joined to a breast hood (3) and defining a longitudinal axis (A), and a suction member, which may be displaced to and fro therein by means of a manually actuable actuation lever (15), preferably a double armed lever (15) pivoted in a middle region (at 19), a restoring device being preferably provided for said actuation device (15) for returning it into a predetermined initial position, wherein said suction member is formed by a membrane (10) of varying thickness, which includes a portion (10') that extends transversely to said displacement space (9) and which is movable with respect to the wall (9') and, in its condition expanded through said displacement space (9), extends also approximately in peripheral direction of said wall (9'), thus being approximately cup-shaped and being reciprocable free of friction within said displacement space (9) by said actuation device (15), **characterised in that** said varying thickness of said membrane (10) comprises the minimum thickness in the centre region (10') and the maximum thickness in the region of its edge (11).

2. Milk pump as claimed in claim 1, **characterised in that** a guiding element (16), attached to said lever (15), is fixed to said transversely extending membrane portion (10') in said centre region, particularly in its centre, and that, as is preferred, said, preferably disk-shaped, guiding element (16) with respect to the smallest transverse dimension (a) of said displacement space (9) is smaller by at least 15%, preferably by at least 20%, particularly by at least 30%, for example by about 35%.

3. Milk pump as claimed in claim 1 or 2, **characterised in that** said displacement space (9) has a straight longitudinal axis (A) and/or straight extending wall, but which optionally diverges from said opening (7') away.

4. Milk pump as claimed in any of the preceding claims, **characterised in that** said displacement space (9) has a cross-section which differs from a cylindrical cross-section, and has in particular a four-cornered cross-section with rounded corners.

5. Milk pump as claimed in any of the preceding claims, **characterised in that** said cup-shaped membrane (10) is about pot-shaped having a bottom surface (10') and at least one peripheral surface (10").

6. Milk pump as claimed in any of claims 1 to 4, **characterised in that** said cup-shaped membrane (10) is at least approximately in the shape of a spherical cap, thus forming said restoring device by this shape.

7. Milk pump as claimed in claim 6, **characterised in that** said membrane (10) receiving displacement space (9) has a cross-section about in the shape of a spherical cap, and that preferably the curvatures of the spherical caps of both parts are substantially equally dimensioned.

8. Milk pump as claimed in any of the preceding claims, **characterised in that** the thickness of said membrane (10) varies in the direction of said longitudinal axis (A).

9. Milk pump as claimed in claim 8, **characterised in that,** particularly in the case of a membrane in the shape of a spherical cap, said centre portion, which is connected to said actuation device (15), said membrane is thinner tan at its edge (11), and that the thickness is preferably continuously varying.

## Revendications

1. Tire-lait comprenant une unité de pompe à main (1) au-dessus d'une ouverture (7'), qui conduit à un récipient (5), ladite unité (1) incluant une chambre d'aspiration (9), qui est sensiblement enfermée par au moins une paroi (9') et raccordée ou capable d'être raccordée à une pièce de mis au sein (3) tout en définissant un axe longitudinal (A), et un organe d'aspiration, qui peut y effectuer un mouvement de vaet-vient au moyen d'un levier d'actionnement (15) à actionner manuellement, de préférence d'un levier intermobile logé d'une façon pivotante dans une zone médiane (chez 19), un arrangement de rappel pour rappeler le dispositif d'actionnement (15) dans une position initiale prédéterminée étant préférablement prévu, dans lequel l'organe d'aspiration est formé par une membrane (10) mobile à l'intérieur de la chambre d'aspiration (9), qui comprend une section (10') s'étendant transversalement à la chambre d'aspiration (9) et, en état étendu à travers la chambre d'aspiration (9), approximativement aussi en direction périphérique de la paroi (9'), et par conséquent étant approximativement en forme de coupe et présentant des variations d'épaisseur, ladite membrane étant capable d'être actionnée d'un mouvement alternatif dans la chambre d'aspiration par le dispositif d'actionnement, **caractérisé en ce, que** les variations d'épaisseur de la membrane (10) présentent l'épaisseur minimale dans la zone médiane (10') et l'épaisseur maximale dans la zone du bord (11).

2. Tire-lait selon la revendication 1, **caractérisé en ce, que** dans la zone médiane un élément de guidage (16) est fixé à celle section de membrane (10'), qui s'étend transversalement à la chambre d'aspiration, particulièrement au centre de celle-ci, et est raccordé au levier (15), et que de préférence l'élément de guidage (16), préférablement en forme de disque, en comparaison avec la dimension transversale la plus petite (a) de la chambre d'aspiration (9) est plus petit par au moins 15%, préférablement par au moins 20%, particulièrement par au moins 30% et est, par exemple plus petit par environ 35%.

3. Tire-lait selon la revendication 1 ou 2, **caractérisé en ce, que** la chambre d'aspiration (9) a un axe longitudinal (A) rectiligne et/ou une paroi (9'), qui s'étend d'une façon rectiligne, ladite paroi, le cas échéant, divergeant en s'éloignant de ladite ouverture (7').

4. Tire-lait selon une quelconque des revendications précédentes, **caractérisé en ce, que** la chambre d'aspiration (9) a une coupe transversale, qui diffère d'une coupe transversale cylindrique, étant en particulier une coupe transversale tetragone ayant des coins arrondis.

5. Tire-lait selon une quelconque des revendications précédentes, **caractérisé en ce, que** la membrane en forme de coupe (10) est formée approximativement en forme de pot ayant une surface de fond (10') et au moins une surface périphérique (10").

6. Tire-lait selon une quelconque des revendications 1 à 4, **caractérisé en ce, que** la membrane en forme de coupe (10) est formée au moins approximativement en forme de calotte, ainsi formant elle-même avec cette forme l'arrangement de rappel.

7. Tire-lait selon la revendication 6, **caractérisé en ce, que** la chambre d'aspiration (9) a une coupe transversal approximativement en forme de calotte contenant la membrane (10), et que de préférence la courbure des calottes des deux parties sont sensiblement également courbées.

8. Tire-lait selon une quelconque des revendications précédentes, **caractérisé en ce, que** l'épaisseur de la membrane (10) varie en direction de son axe longitudinal (A).

9. Tire-lait selon la revendication 8, **caractérisé en ce, que,** de préférence en cas d'une membrane (10) en forme de calotte, la section médiane (10') de la membrane, qui est raccordée au dispositif d'actionnement (15), est plus mince, qu'à son bord (11), et présente, préférablement, une variation d'épaisseur sensiblement continuelle.
